# EUROPEAN PATENT APPLICATION

(11) **EP 1 598 019 A2**
(43) Date of publication of application: **23.11.2005**
(21) Application number: 05010979.2
(22) Date of filing: 20.05.2005
(51) Int. Cl.: A61B 17/12, A61B 17/04

(54) **Treatment system for living tissues**

(30) Priority: 20.05.2004 US 572968 P
(71) Applicant: Olympus Corporation, Shibuya-ku, Tokyo (JP)
(72) Inventor: Sakamoto, Yuji c/o Olympus Corporation, Hachioji-shi Tokyo (JP); Yamamoto, Tetsuya c/o Olympus Corporation, Hachioji-shi Tokyo (JP); Suzuki, Keita c/o Olympus Corporation, Hachioji-shi Tokyo (JP)
(74) Representative: von Hellfeld, Axel

(57) **Abstract**

A medical treatment device (12) includes a ligating member (22), a stopper (26) and a ligation releasing member (30). The ligating member has a distal end portion and a proximal end portion, that ligates biological tissue. The stopper is provided to be movable forward or backward with respect to the ligating member, and stoppable by friction on the ligating member to maintain the biological tissue in a ligated state by the ligating member. The ligation releasing member is provided on the ligating member to release the ligation state between the ligating member and the stopper by moving the ligating member to the distal end side with respect to the stopper.

## Description

The present invention relates to a treatment system for living tissues, which is designed for use in combination with an endoscope and another type of an instrument to perform an endoscopic treatment such as suture and ligation on the living tissues in the body.

U.S. Patent Application Publication No. 2003/0236535A1 (hereinafter referred to as "publication") discloses a ligation apparatus for use in endoscopic treatment, i.e., the apparatus that a surgeon uses to suture or ligate the living tissues in the body while observing the tissues through an endoscope. The apparatus disclosed in the publication enables the surgeon to suture or ligate the tissues by passing the ligature attached to a holding member, through the tissues. To release the tissues from the sutured or ligated state, the surgeon cuts the ligature, or grasps and removes the holding member from the tissues. The tissues may not be sutured or ligated at desired parts. In this case, the surgeon cuts the ligature to release the tissues from the sutured or ligated state. To cut the ligature the surgeon uses, for example, surgical scissors, while observing the tissues through an endoscope. Otherwise, the surgeon may grasp the holding member with a grasping forceps or the like and then remove the holding member from the tissues, while observing the tissues through the endoscope.

With the method disclosed in the above-identified reference, it is very complicated to operate the forceps and difficult to cut the ligature without damaging the living tissue when the ligature is buried in the tissue.

There is another method in which grasping forceps are used endoscopically to grasp tissue and then the stopper is removed. However, with this method, it is very complicated to operate the forceps when the stopper has such a shape that is difficult to grasp.

Further, it is very complicated and difficult to release ligation by applying a force larger than the stopper force to the stopper or ligature which is fixed with a force necessary for the ligation.

The present invention has been proposed to solve the above-described drawbacks of the conventional techniques, and an object thereof is to provide a medical treatment device capable of ligating living tissue under endoscopic observation, and capable of releasing ligation of living tissue by a simple operation, as well as a treatment system for living tissue.

According to an aspect of this invention, there is provided a medical treatment device includes a ligating member, a stopper and a ligation releasing member. The ligating member has a distal end portion and a proximal end portion, that ligates biological tissue. The stopper is provided to be movable forward or backward with respect to the ligating member, and stoppable by friction on the ligating member to maintain the biological tissue in a ligated state by the ligating member. The ligation releasing member is provided on the ligating member to release the ligation state between the ligating member and the stopper by moving the ligating member to the distal end side with respect to the stopper.

This summary of the invention does not necessarily describe all necessary features so that the invention may also be a sub-combination of these described features.

The invention can be more fully understood from the following detailed description when taken in conjunction with the accompanying drawings, in which:
FIG. 1 is a schematic sectional view of a treatment system for living tissues, according to a first embodiment of the invention, showing the ligation apparatus and the ligation instrument coupled to the apparatus;
FIG. 2 is a schematic perspective view of the ligation apparatus according to the first embodiment;
FIG. 3 is a schematic sectional view of the treatment system according to the first embodiment, depicting a ligature wrapped around the tissue to be treated;
FIG. 4 is a schematic perspective view of the ligature wrapped around the living tissue by using the ligation apparatus of the treatment system according to the first embodiment, the ligature cut at the parts protruding from a silicone tube;
FIG. 5 is a schematic perspective view of the ligature which is wrapped around the living tissue by using the ligation apparatus of the treatment system according to the first embodiment, illustrating a flexible wire that is held with a grasping forceps and pulled to release the tissue from the ligated state;
FIG. 6 is a schematic top view showing how the tissue is released from the ligated state as the flexible wire is pulled and one of the ends of the ligature is thereby pulled out of the silicone tube in the treatment system according to the first embodiment;
FIG. 7 is a schematic perspective view illustrating how the flexible wire held with the grasping forceps is pulled in the treatment system according to the first embodiment, in order to remove the ligature from the tissue;
FIG. 8 is a schematic perspective view of the ligation apparatus incorporated in a treatment system for living tissues, according to a second embodiment of the invention;
FIG. 9 is a schematic perspective view of the ligation apparatus provided in a treatment system for living tissues, according to a third embodiment of the invention;
FIG. 10 is a schematic perspective view of the ligation apparatus used in a treatment system for living tissues, according to a fourth embodiment of the invention;
FIG. 11 is a schematic perspective view of the ligation apparatus provided in a treatment system for living tissues, according to a fifth embodiment of the invention;
FIG. 12 is a schematic perspective view of the ligation apparatus incorporated in a treatment system for living tissues, according to a sixth embodiment of the invention;
FIG. 13 is a schematic sectional view illustrating a silicone tube holding a ligature by virtue of friction, in the ligation apparatus of the treatment system according to the sixth embodiment;
FIG. 14 is a schematic sectional view showing the silicone tube in which the ligature can slide, in the ligation apparatus of the treatment system according to the sixth embodiment;
FIG. 15 is a schematic perspective view of the ligation apparatus incorporated in a treatment system for living tissues, according to a seventh embodiment of the invention;
FIG. 16 is a schematic perspective view, illustrating how a flexible wire is held and pulled with a grasping forceps after the tissue has been ligated, thereby to split a silicone tube toward its proximal end, in the treatment system according to the seventh embodiment;
FIG. 17 is a schematic perspective view, showing how the silicone tube is split to release the ligature with the flexible wire in the treatment system according to the seventh embodiment;
FIG. 18 is a schematic perspective view depicting the ligation apparatus used in a treatment system for living tissues, according to an eighth embodiment of the invention; and
FIG. 19 is a schematic perspective view, illustrating how one of the three silicone tubes is removed from the ligature after the tissue has been ligated, thereby to adjust the ligated condition of the tissue, in the treatment system according to the eighth embodiment.

Preferred embodiments of the invention will be described, with reference to the accompanying drawings.

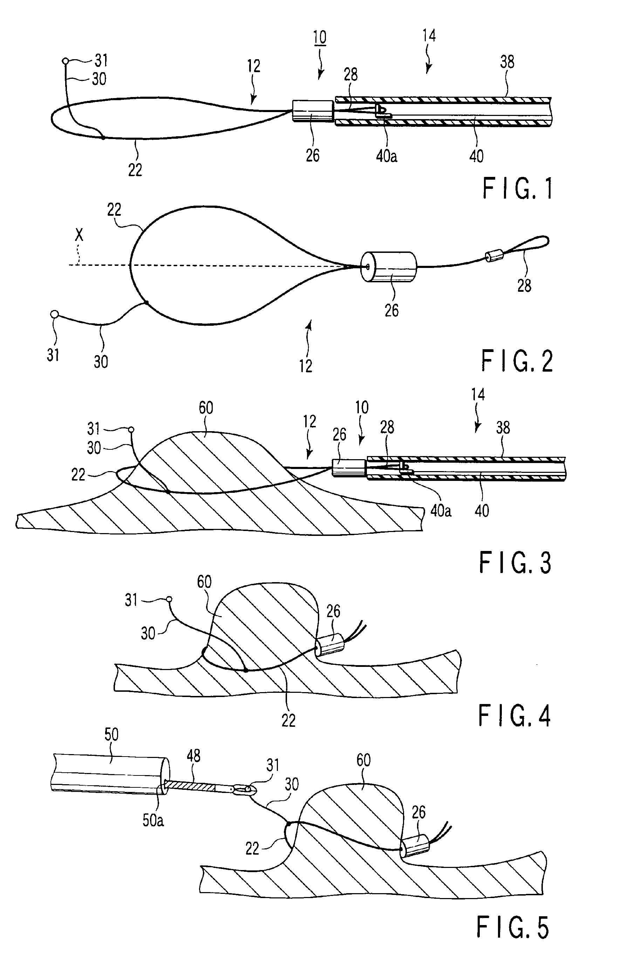

First, the first embodiment will be described with reference to FIGS. 1 to 7.

The treatment system 10 for living tissues, shown in FIG. 1, is a system designed to ligate living tissues. The system 10 may be endoscopically used, or in combination with an endoscope, to ligate a polyp or the like before removing it, thereby to control bleeding. The system 10 comprises a ligation apparatus 12 and a ligation instrument 14. The instrument 14 is used in combination with the ligation apparatus 12.

As FIG. 2 shows, the ligation apparatus 12 comprises a ligature (ligating member) 22, a silicone tube (stopper) 26, a proximal loop 28, and a flexible wire (ligation-releasing member) 30. The proximal loop 28 is formed at the proximal ends of the ligature 22.

The ligature 22 is provided in the form of a loop. Therefore, its two end portions pass through the silicone tube 26. The silicone tube 26 is held, by virtue of a frictional force, on any desired part of the ligature 22. The tube 26 can be moved when a force greater than a prescribed value is applied along the ligature 22. Thus, the silicone tube 26 performs the function of a stopper that holds the ligature 22 at a desired position with respect to the tissue that is to be treated.

The flexible wire 30 is connected to the ligature 22, at a position close to the distal end of the ligature 22 and deviated from the axis X of the lumen into which the ligature 22 should be inserted. One flexible wire is used in this embodiment. Nonetheless, two, three or more flexible wires may be connected to the ligature 22. It is desirable to connect one or two flexible wires to the ligature 22. If too many flexible wires are connected to the ligature 22, they may entangle while the ligation apparatus 12 remains in the body. The flexible wire 30 may be replaced by a member of a different shape, such as a band.

The flexible wire 30 can be made of any material that is flexible and strong enough to withstand a pulling force applied to release the tissue from a ligated state. It may be made of the same material as suture thread, e.g., drawn polyamide synthetic fiber, polypropylene, polyethyleneterephthalate, polytetrafluoroethylene. Alternatively, it may be made of bioabsorbable material such as polyglycolic acid. The wire may be either a monofilament one or a twisted thread. The flexible wire 30 is colored not similar to the living tissues, not white, red or yellow, and different in color from the ligature 22. Hence, the surgeon can well distinguish the wire 30 when he or she sees it through the endoscope.

As FIG. 1 depicts, the ligation instrument 14 comprises a ligation sheath 38, a hook wire 40, and an operation handle (not shown). The hook wire 40 is connected, at its distal end, to a hook 40a, and extends through the ligation sheath 38. The hook 40a catches the proximal loop 28 of the ligature 22. The operation handle is provided at the proximal end of the ligation sheath 38 and that of the hook wire 40.

How this embodiment is operated will be explained. Note that the embodiment is endoscopically operated, though the operation may not be explained without referring to the endoscope used. Here, it will be described how the embodiment is manipulated to ligate the tissue 60 to be treated and to release the tissue 60 from the ligated state. First, it will be described how to ligate the tissue 60.

As shown in FIG. 1, the ligation instrument 14 is coupled to the ligation apparatus 12 (see FIG. 2). The proximal loop 28 of the ligation apparatus 12 is set into engagement with the hook 40a fastened to the hook wire 40 of the ligation instrument 14. The silicone tube 26 of the ligation apparatus 12 is moved until it abuts on the distal end of the ligation sheath 38 of the ligation instrument 14.

As FIG. 3 shows, the looped ligature 22 of the treatment system 10 is rapped around the tissue 60 that is to be treated. When the hook wire 40 of the ligation instrument 14 is pulled with respect to the ligation sheath 38, the silicone tube 26 is pushed at the distal end of the ligation sheath 38. As a result, the silicone tube 26 moves toward the distal end of the ligature 22.

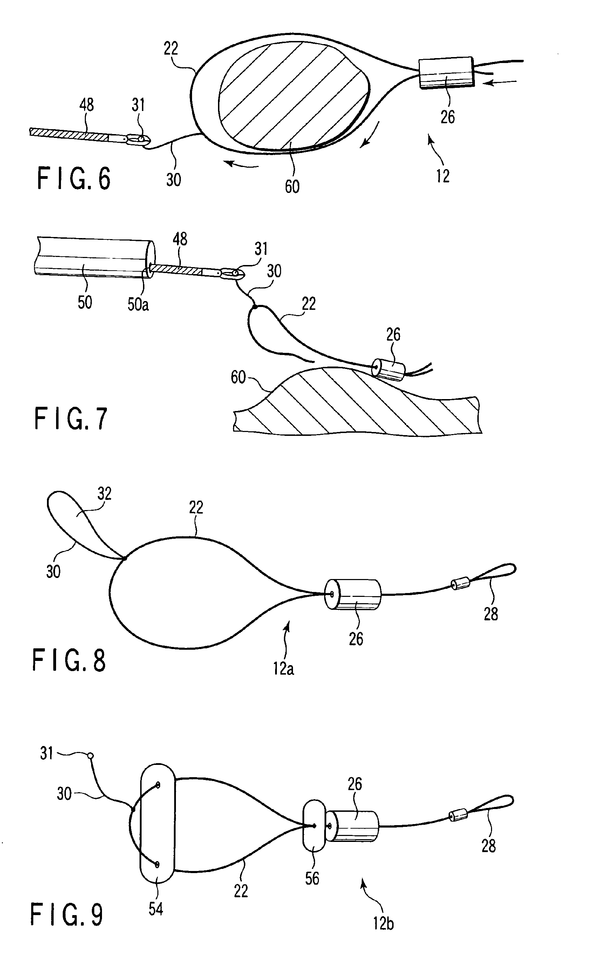

As FIG. 4 depicts, the ligature 22 squeezes the tissue 60 as the loop of the ligature 22 becomes smaller. The tissue 60 is thereby ligated. The silicone tube 26 is prevented from moving relative to the ligature 22, by virtue of a frictional force. The tissue 60 therefore remains ligated. After the tissue 60 has been thus ligated, the parts of the ligature 22, which protrude from the proximal end of the silicone tube 26, are cut off.

It will be explained how the tissue 60 is released from the ligated state.

As FIG. 5 shows, an endoscope 50 having a channel 50a that can guide a grasping forceps 48 is used to release the tissue 60 from the ligated state. The grasping forceps 48 extending through the channel 50a holds the flexible wire 30. The forceps 48 is pulled, thus pulling the flexible wire 30 with a force greater than the frictional force that the silicone tube 26 exerts on the wire 30.

As is illustrated in FIG. 6, the flexible wire 30 is connected to the distal part of the looped ligature 22, at a position deviated from the axis X of the lumen (see FIG. 2). When the flexible wire 30 is pulled, one of the end portions of the ligature 22, which extend through the silicone tube 26, is pulled from the silicone tube 26. As a result, the diameter of the looped ligature 22 increases, and the ligature 22 is removed from the tissue 60. When the flexible wire 30 is pulled by manipulating the gasping forceps 48, the portions of the ligature 22 is pulled out of the silicone tube 26, one after the other. Hence, the ligature 22 can be released from the silicone tube 26 with a smaller force than in the case where both end portions are pulled at the same time.

As FIG. 7 shows, the ligature 22 and the silicone tube 26 are moved away from the tissue, while the flexible wire 30 remains held by the grasping forceps 48.

The embodiment described above is advantageous in the following respects.

To release the tissue 60 from the ligated state, the flexible wire 30 is held with the grasping forceps 48. It is easier for the surgeon to hold the flexible wire 30 than to hold the ligature 22 directly, while observing the tissue through the endoscope 50. This prevents any damage to the tissue 60. Further, the surgeon would not fail to hold the flexible wire 30 with the grasping forceps 48, because the flexible wire 30 has a loop 31 at the distal end.

Once the flexible wire 30 has been held with the grasping forceps 48, the ligature 22 can be removed from the tissue 60 and pulled from the silicone tube 26, in whichever direction the flexible wire 30 is pulled.

The second embodiment of the invention will be described, with reference to FIG. 8. The embodiment is a modification of the first embodiment. The components identical to those of the first embodiment are designated at the same reference numerals and will not be described in detail.

As can be seen from FIG. 8, the ligation apparatus 12a according to the embodiment differs from the ligation apparatus 12 (see FIG. 2) according to the first embodiment in that the flexible wire 30 has a looped part 32 and is connected to the ligature 22.

When the apparatus 12a is used, it performs the same operation as the first embodiment and can achieve the same result as the first embodiment. In addition, the flexible wire 30 can be easily held with, for example, a grasping forceps 48, because the flexible wire 30 has a looped part 32. Thanks to the looped part 32, the grasping forceps 48 can hold the flexible wire 30 at two parts. Hence, the flexible wire 30 can be pulled by using the grasping forceps 48, with a greater force than when the forceps 48 holds the wire 30 at only one part (see FIG. 2).

The third embodiment will be described, with reference to FIG. 9. The embodiment is a modification of the first embodiment. The components identical to those of the first embodiment are designated at the same reference numerals and will not be described in detail.

As can be seen from FIG. 9, the ligation apparatus 12b according to the embodiment differs from the ligation apparatus 12 (see FIG. 2) according to the first embodiment in several respects.

A distal pledget 54 is arranged near the distal end of the looped ligature 22 and positioned symmetrical with respect to the axis X of the lumen of the silicone tube 26 (see FIG. 2). The distal pledget 54 has a pair of holes, which are symmetrical to each other with respect to axis X of the lumen of the silicone tube 26. Two parts of the ligature 22 pass through these holes, respectively.

A proximal pledget 56 is provided between the distal pledget 54 and the silicone tube 26 and located near the silicone tube 26. The proximal pledget 56 has a hole made in the center part. Two parts of the looped ligature 22 pass through this hole. Hence, the tissue 60 can be ligated, while being held between the distal pledget 54 and the proximal pledget 56.

When the apparatus 12b is used, it performs the same operation as the first embodiment and can achieve the same result as the first embodiment. In addition, the flexible wire 30 can be prevented from contacting the tissue 60 when the tissue 60 is ligated, because the distal pledget 54 contacts the tissue 60 at a large area. Thus, the flexible wire 30 can be easily grasped with the grasping forceps 48 in order to remove the ligature 22 from the tissue 60 that has been ligated by using the ligation apparatus 12b.

Once the tissue 60 has been ligated, the distal pledget 54 contacts the tissue 60 at a large area. This reliably prevents the ligature 22 from being embedded in the tissue 22. Further, the proximal pledget 56 contacts the tissue 60 at a large area once the tissue 60 has been ligated. This also reliably prevents the ligature 22 from being embedded in the tissue 22.

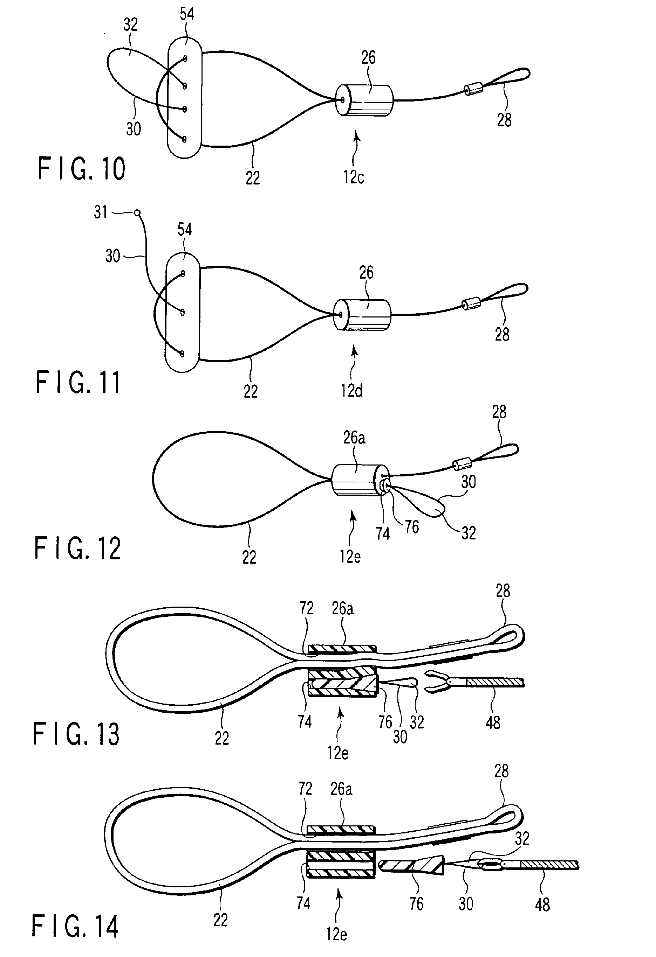

The fourth embodiment will be described, with reference to FIG. 10. The embodiment is a modification of the third embodiment. The components identical to those of the third embodiment are denoted at the same reference numerals and will not be described in detail.

As can be understood from FIG. 10, the ligation apparatus 12c according to the embodiment differs from the ligation apparatus 12b (see FIG. 9) according to the third embodiment in that the flexible wire 30 has a looped part 32 that is connected to the distal pledget 54. The flexible wire 30 can slide with respect to the distal pledget 54. The ligation apparatus 12c has no proximal pledgets 56.

When this apparatus 12c is used, it performs the same operation as the third embodiment and can achieve the same result as the third embodiment. In addition, the flexible wire 30 can be easily held with, for example, a grasping forceps 48, because the flexible wire 30 has a looped part 32. Thanks to the looped part 32, the grasping forceps 48 can hold the flexible wire 30 at two parts. Hence, the flexible wire 30 can be pulled by using the grasping forceps 48, with a greater force than when the forceps 48 holds the wire 30 at only one part (see FIG. 9).

Moreover, that part of the flexible wire 30, which is connected to the distal pledget 54, would not interfere with the distal pledget 54 when the tissue 60 is ligated. The tissue 60 can therefore be ligated more reliably.

The fifth embodiment will be described, with reference to FIG. 11. The embodiment is a modification of the fourth embodiment. The components identical to those of the fourth embodiment are denoted at the same reference numerals and will not be described in detail.

As can be seen from FIG. 11, the ligation apparatus 12d according to the embodiment differs from the ligation apparatus 12c (see FIG. 10) according to the fourth embodiment in that a flexible wire 30 having an expanded part 31 is connected to the distal pledget 54.

When this apparatus 12d is used, it performs the same operation as the fourth embodiment and can achieve the same result as the fourth embodiment. In addition, the flexible wire 30 is so shaped not to occupy much space, when the apparatus 12d is endoscopically left in a body cavity. This prevents the flexible wire 30 from entangling with the food being swallowed, the endoscope 50 being removed, or the grasping forceps 48 being removed.

The sixth embodiment will be described, with reference to FIGS. 12 to 14. The embodiment is a modification of the first embodiment. The components identical to those of the first embodiment are designated at the same reference numerals and will not be described in detail.

As can be seen from FIG. 12, the ligation apparatus 12e according to this embodiment differs from the ligation apparatus 12 (see FIG. 2) according to the first embodiment in some respects.

As FIGS. 13 and 14 show, the silicone tube 26a according to the embodiment has two lumens, i.e., a ligature-guiding lumen 72 and a plug-receiving lumen 74. The lumens 72 and 74 are arranged adjacent to each other and extend parallel to the axis X (see FIG. 2) of the silicone tube 26.

A ligature 22 passes through the ligature-guiding lumen 72. The ligature-guiding lumen 72 has an inside diameter large, allowing the ligature 22 to slide smoothly.

A plug 76 has been pushed into the plug-receiving lumen 74. The plug-receiving lumen 74 has an inside diameter small than the diameter of the plug 76. Nonetheless, the plug 76 can be inserted into the plug-receiving lumen 74. To the proximal end of the plug 76 there is connected to a flexible wire 30 that has a looped part 32.

How the embodiment is operated will be explained. First, it will be described how the embodiment ligates tissue 60 to be treated. The ligation apparatus 12e shown in FIG. 12, which has the plug 76 inserted in the plug-receiving lumen 74, is used in place of the apparatus 12 (see FIG. 2) according to the first embodiment.

As illustrated in FIG. 3, the hook wire 40 of the ligation instrument 14 is pulled with respect to the ligation sheath 38. The silicone tube 26 is therefore pushed at the distal end and moved toward the distal end of the ligature 22 along the ligature 22. As a result, the apparatus 12e shown in FIG. 13 gradually reduces the diameter of the looped part of the ligature 22, clamping the tissue 60 with the ligature 22. Thus, the apparatus 12e ligates the tissue 60.

As FIG. 4 shows, the parts of the ligature 22, which protrude from the proximal end of the silicone tube 26, are cut off after the tissue 60 has been thus ligated.

It will be explained how the tissue 60 is released from the ligated state.

To release the tissue 60 from the ligated state, the endoscope 50 (see FIG. 5) is used, which has a channel 50a that can guide a grasping forceps 48. The flexible wire 30 is held with the grasping forceps 48 that extends through the channel 50a. Then, the flexible wire 30 is pulled with a force greater than the frictional force that retains the plug 76 in the plug-receiving lumen 74. As a result, the ligature-guiding lumen 72 expands, having its inside diameter increases. The ligature 22 can now slide. That is, there is no long a ligature-holding force resulting from the friction between the silicone tube 26 and the ligature 22. Either the silicone tube 26 or the ligature 22 is held with the grasping forceps 48, the tube 26 is move away from the ligature 22 or vice versa. The tissue 60 is thereby released from the ligated state.

The embodiment described above is advantageous in some respects. When this apparatus 12e is used, it performs the same operation as the first embodiment and can achieve the same result as the first embodiment. In addition, the looped part of the ligature 22 can easily expand because the ligature 22 smoothly moves in the ligature-guiding lumen 72. Because of this, it is easy to release the tissue 60 from the ligated state.

While the tissue 60 is being released from the ligated state, no tension is applied on the ligature 22 or the silicone tube 26. The ligature 22 can be removed from the tissue 60, not influencing the tissue 60 at all.

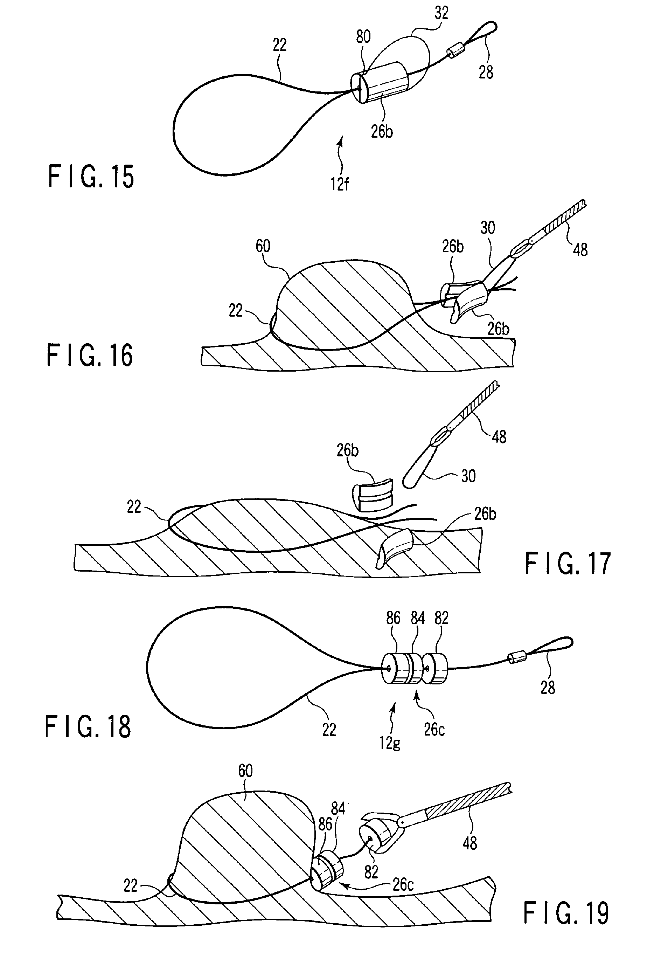

The seventh embodiment will be described, with reference to FIGS. 15 to 17. The embodiment is a modification of the first embodiment. The components identical to those of the first embodiment are designated at the same reference numerals and will not be described in detail.

As FIG. 15 may suggest, the ligation apparatus 12e according to this embodiment differs from the ligation apparatus 12 (see FIG. 2) according to the first embodiment in some respects.

As FIGS. 15 to 17 depict, the silicone tube 26b according to the embodiment has a slit 80 that extends at right angles to the axis X (see FIG. 2) of the lumen. A flexible wire 30 having a looped part 32 passes through the slit 80.

How the embodiment is operated will be explained. To ligate the tissue 60 to be treated, it is operated in the same manner as the first embodiment. Therefore, it will be described how the embodiment is operated to release the tissue 60 from the ligated state.

To release the tissue 60 from the ligated state, the endoscope 50 is used, which has a channel 50a that can guide a grasping forceps 48. As shown in FIG. 16, the flexible wire 30 is held with the grasping forceps 48 that extends through the channel 50a. Then, the flexible wire 30 is held with a grasping forceps 48 that extends through the channel 50a. As the flexible wire 30 is pulled, the silicone tube 26b is split, from the slit 80 toward the proximal end of the silicone tube 26b.

When the flexible wire 30 is further pulled, the silicone tube 26b splits into two parts as illustrated in FIG. 17. As a result, the ligature 22 can no longer remain looped. Hence, the ligature 22 is easily removed from the tissue 60, releasing the tissue 60 from the ligated state.

The embodiment described above is advantageous in some respects. When the apparatus 12f is used, it performs the same operation as the first embodiment and can achieve the same result as the first embodiment. In addition, the silicone tube 26b can slits into two parts along the axis X of the lumen to release the tissue 60 from the ligated state, when the flexible wire 30 is held with the grasping forceps 48 and pulled toward its proximal end. Thus, the tissue 60 can easily be released from the ligated state. Even if the ligature 22 hardly slides on the silicone tube 26b and the friction between the ligature 22 and the tube 26b is high, the ligature 22 that was held by the silicone tube 26b can easily be removed from the tissue 60.

The eighth embodiment will be described, with reference to FIGS. 18 to 19. The embodiment is a modification of the first embodiment. The components identical to those of the first embodiment are designated at the same reference numerals and will not be described in detail.

As seen from FIG. 18, the ligation apparatus 12g according to this embodiment differs from the ligation apparatus 12 (see FIG. 2) according to the first embodiment in that a silicone tube 26c is used in place of the silicone tube 26. The tube 26c has the same length as the silicone tube 26, but is composed of three silicone tubes 82, 84 and 86. In other words, the tube 26c is divided into three parts, along lines perpendicular to the longitudinal axis.

How the embodiment is operated will be explained. To ligate the tissue 60 to be treated, it is operated in the same manner as the first embodiment. Therefore, it will be described how the embodiment is operated to release the tissue 60 from the ligated state.

To release the tissue 60 from the ligated state, the endoscope 50 is used as shown in FIG. 19, which has a channel 50a that can guide a grasping forceps 48. The first silicone tube 82 is held with the grasping forceps 48 that extends through the channel 50a. Then, the first silicone tube 82 is pulled toward the proximal end of the ligature 22. Similarly, the second silicone tube 84 and the third silicone tube 86 are pulled toward the proximal end of the ligature 22, by using the grasping forceps 48. Since the total length of the first to third silicone tube 82, 84 and 86 is equal to the silicone tube 26 of the first embodiment, the friction between the ligature 22 and each of the silicone tubes 82, 84 and 86 is smaller than the frictional force between the ligature 22 and the silicone tube 26 of the first embodiment. The silicone tubes 82, 84 and 86 can therefore be moved toward the proximal end of the ligature 22. This makes it easy to remove the ligature 22 from the tissue 60 and, hence, to release the tissue 60 from the ligated state. As long as the ligature 22 extends through the first to third silicone tubes 82, 84 and 86, a desired frictional force of course remains exerted on the ligature 22.

The embodiment described above is advantageous in some respects. When the apparatus 12g is used, it performs the same operation as the first embodiment and can achieve the same result as the first embodiment. In addition, the silicone tubes 82, 84 and 86 can be removed, one by one, from the ligature 22, to release the tissue 60 from the ligated state. Hence, a small grasping force suffices to hold one silicone tube in the process of removing the tube from the ligature 22. The surgeon would not fail to hold each silicone tube with the grasping forceps 48 and can therefore easily release the tissue 60 from the ligated state.

After the tissue 60 has been ligated, one, two or three tubes that constitute the silicone tube 26c may be removed. Thus, the ligating force applied to the tissue 60 can be adjusted.

## Claims

1. A medical treatment device (12) **characterized by** comprising:
a ligating member (22) having a distal end portion and a proximal end portion, that ligates biological tissue;
a stopper (26) provided to be movable forward or backward with respect to the ligating member, and stoppable by friction on the ligating member to maintain the biological tissue in a ligated state by the ligating member; and
a ligation releasing member (30) provided on the ligating member to release the ligation state between the ligating member and the stopper by moving the ligating member to the distal end side with respect to the stopper.

2. The medical treatment device (12) according to claim 1, **characterized in that** the ligating member comprises a ligation releasing member movement limiting portion that limits movement of the ligation releasing member (30) on the ligating member.

3. The medical treatment device (12) according to claim 1 or 2, **characterized in that** the ligation-releasing member (30) includes:
a first end portion equipped with a pledget (14) provided at a position opposite to the stopper (26) to prevent the ligating member (22) from digging into the biological tissue; and
a second end portion located on a side opposite to the first end portion and equipped with a to-be-grasped portion (31, 32) which is grasped when ligation by the ligating member is released.

4. The medical treatment device (12) according to any one of claims 1 to 3, **characterized in that** the ligating member (22) comprises a loop portion provided on a distal end side of the stopper (26) and first and second stopper portions fixed by friction to the stopper (26),
the ligation-releasing member (30) comprises an anchor portion anchored to the loop portion,
and the anchor portion is located at a section such that a distance between the section and the first stopper portion and another distance between the section and the second stopper portion are different from each other.

5. The treatment device (12) according to any one of claims 1 to 4, **characterized in that** the ligation releasing member (30) is a flexible wire material.

6. The treatment device (12) according to claim 5, **characterized in that** the ligation releasing member (30) has an expanded part (31) in at least one portion thereof.

7. The treatment device (12) according to claim 5 or 6, **characterized in that** the ligation releasing member (30) has a loop (32).

8. A medical treatment device (12) **characterized by** comprising:
a ligating member (22) having a distal end portion and a proximal end portion, that ligates biological tissue;
a stopper (26) provided to be movable forward or backward with respect to the ligating member, and stoppable by friction on the ligating member to maintain the biological tissue in a ligated state by the ligating member; and
a force adjusting member (76) provided on the ligating member to change a stopping force of the stopper to the ligating member.

9. The treatment device (12) according to claim 8, **characterized in that** the stopper (26) comprises a first lumen (72) through which the ligating member is pierced and a second lumen (74) configured to hold the force adjusting member (76) when the member is pressed therein, and
the force adjusting member comprises a plug that adjusts the stopping force of the ligating member by deforming the stopper when the force adjusting member is pressed into the second lumen.

10. A medical treatment device (12) **characterized by** comprising:
a ligating member (22) having a distal end portion and a proximal end portion, that ligates biological tissue;
a stopper (26) provided to be movable forward or backward with respect to the ligating member, and stoppable by friction on the ligating member to maintain the biological tissue in a ligated state by the ligating member, the stopper splitting into at least two when releasing the ligation.

11. A treatment system for living tissue, **characterized by** comprising a medical treatment device (12) according to any one of claims 1 to 10, the system further comprising:
a tube-shaped sheath (38) having a distal end configured to abut to the stopper (26);
a hook wire (40) pierced through the sheath and having a hook (40a) configured to hook to a proximal end of the ligation releasing member; and
an operation handle that moves the hook wire forward and backward with respect to the sheath.
